# NEW EUROPEAN PATENT SPECIFICATION

(11) **EP 2 367 521 B2**
(45) Date of publication and mention of the opposition decision: **29.09.2021**
(45) Mention of the grant of the patent: 27.03.2013
(21) Application number: 09763975.1
(22) Date of filing: 04.12.2009
(51) Int. Cl.: A61K 8/26, A61K 8/34, A61K 8/92, A61Q 15/00

(54) **ANTIPERSPIRANT COMPOSITION COMPRISING MICA AND FATTY ALCOHOL WAX**
SCHWEISSHEMMENDE ZUSAMMENSETZUNG ENTHALTEND GLIMMER UND FETTALKOHOLWACHS
COMPOSITION ANTI-TRANSPIRANTE COMPRENANT DU MICA ET UNE CIRE D'ALCOOL GRAS

(30) Priority: 24.12.2008 EP 08022462
(43) Date of publication of application: 28.09.2011
(73) Proprietor: Unilever IP Holdings B.V., 3013 AL Rotterdam (NL); Unilever Global IP Limited, Wirral, CH62 AZD (GB)
(72) Inventor: BRENNAN, Gail, Christine, Merseyside CH63 3JW (GB); BUTTERWORTH, Andrew, Yorkshire LS14 2AR (GB); FERRIER, Lindsay, Karen, Yorkshire LS14 2AR (GB); JONES, Shirley, Yorkshire LS14 2AR (GB); MARRIOTT, Robert, Edward, Merseyside CH63 3JW (GB); WILLIAMS, Jason, Richard, Merseyside CH63 3JW (GB); POLONKA, Jack, CT 06611 (US)
(74) Representative: Potter Clarkson
(86) International application number: PCT/EP2009/066403
(87) International publication number: WO 2010/072545

(56) References cited:
- EP-A1- 2 090 284
- DE-A1-102005 026 034
- US-A- 6 103 250
- US-A1- 2002 182 233
- US-A1- 2004 091 439
- US-A1- 2006 051 305
- US-A1- 2008 152 608
- Engelhard Flamenco Orange 320C datasheet

## Description

The present invention relates to antiperspirant compositions and more particularly to compositions that are gelled by a fatty alcohol wax.

### Background and Prior Art

For many years, humans have employed cosmetic methods, sometimes alternative referred to as non-therapeutic methods, to prevent or at least ameliorate bodily functions which society at the time under consideration considers to be unsightly or otherwise undesirable. These methods have included controlling the appearance of sweat by topical application of an active which prevents egress of sweat from the eccrine glands. The active can be applied cosmetically and topically to the skin, broadly speaking, by one of two methods. Different consumers prefer one method or the other. In one method, sometimes called a contact method, a composition is wiped across the surface of the skin, depositing a fraction of the composition as it passes. In the second method, sometimes called the non-contact method, the composition is sprayed from a dispenser held proximate to the skin, often in the region of 10 to 20cms. The spray can be developed by mechanical means of generating pressure on the contents of the dispenser, such as a pump or a squeezable sidewall or by internally generated pressure arising from a fraction of a liquefied propellant volatilising, the dispenser commonly being called an aerosol.

There are broadly speaking two classes of contact compositions, one of which is liquid and usually applied using a roll-on dispenser or possibly absorbed into or onto a wipe, and in the second of which the antiperspirant active is distributed within a carrier liquid that forms a continuous phase that has been gelled. In one variation, the carrier fluid comprises a solvent for the antiperspirant and in a second variation, the antiperspirant remains a particulate solid that is suspended in an oil, usually a blend of oils.

Many different materials have been proposed as gellant for a continuous oil phase, including waxes, small molecule gelling agents and polymers, They each have their advantages and of them, one of the most popular class of gellant has comprised waxes, partly at least due to their ready availability and ease of processing, including in particular linear fatty alcohol wax gellants. A gelled antiperspirant composition is applied topically to skin by wiping it across and in contact with the skin, thereby depositing on the skin a thin film.

The nature of the film depends to a significant extent on the gellant that is employed. Although wax fatty alcohols have been employed as gellant for many years, and are effective for the purpose of gelling, the resultant product is rather ineffective at improving the visual appearance of skin, and in particular underarm skin, to which the composition has been applied. This is noticeable, for example, when the composition contains other ingredients intended to improve the quality of skin, or at least to ameliorate the demoisturising effect of an astringent aluminium-containing antiperspirant active. Such ameliorating materials include, for example, di or polyhydric humectants and/or a triglyceride oil. Such visual improvements include the tone, radiance and/or smoothness of skin, assessed visually and/or the visibility of skin imperfections. Such attributes are of particular importance in relation to skin that has been shaven or plucked and can arise as a person ages.

US 6 103 250 A (BRIEVA HERNANDO [US] ET AL) 15 August 2000 (2000-08-15) discloses (ex. 10) a cosmetic antiperspirant composition comprising:
- 5 to 30% by weight of an astringent antiperspirant salt: Al Zr tetrahydrochlorhydrex
- 40 to 80% by weight of a continuous liquid phase comprising at least one water - immiscible oil (cyclomethicone)
- 5 to 30% by weight of a gellant for the carrier liquid selected from fatty alcohol waxes: stearyl alcohol, octyldodecanol.

### Object of the present invention

It is an object of at least some embodiments of the present invention to devise antiperspirant compositions that improve the skin appearance of skin to which a fatty alcohol gelled composition has been applied.

It is an object of certain embodiments to devise antiperspirant compositions that contain a moisturiser and/or a triglyceride oil that improve the skin appearance of skin to which a fatty alcohol gelled composition has been applied.

Other and further objects may become apparent in the subsequent text herein.

### Brief summary of the present invention

According to one aspect of the present invention, there is provided a cosmetic antiperspirant composition comprising from 5 to 30% by weight of an astringent antiperspirant salt, from 40 to 80% by weight of a continuous liquid phase comprising at least one water-immiscible oil and from 10 to 30% by weight of a gellant for the carrier liquid selected from fatty alcohol waxes, and containing at least 0.1 % by weight of at least one aliphatic dihydric or trihydric moisturiser having a molecular weight of not greater than 620 and/or at least 0.1 % by weight of a triglyceride oil, which composition further containing at least 0.25% by weight of a mica pigment dispersed within the continuous liquid phase.

By dispersing the mica pigment within continuous oil phase, the composition enjoys increased lustre and brightness and the matte appearance caused by the wax gellant is to some extent counteracted. Indeed, by adopting the compositions according to the present invention, a balance can be struck between matte and gloss effects that improves the visual appearance of for example underarm skin, improving its tone and/or radiance and/or visual smoothness without excessively increasing white visible deposits and/or shininess.

According to a second aspect of the present invention there is provided a process for the manufacture of a composition according to the first aspect in which in one step from 40 to 80 parts of oil are mixed with from 8 to 30 parts by weight of the fatty alcohol gellant and the oil and the gellant are heated separately or together to a temperature in excess of 70°C at which the gellant is melted, the oil containing at least 0.1 parts by weight of a di or polyhydric humectant having a molecular weight of from 200 to 620 and/or at least 0.1 part by weight of a triglyceride oil, to form a fluid mixture, mixing the fluid mixture with particulate astringent antiperspirant active and dispersing within the fluid mixture or within the oil at least 0.25 parts by weight of a mica pigment, agitating the mixture and cooling or allowing it to cool to a temperature not higher than 5°C above its normal solidification temperature and charging the mixture into a dispensing applicator or mould.

By retaining the mica pigment dispersed within the continuous oil phase, the pigment is distributed throughout the phase and acts to counteract the matte finish of the gellant that is likewise distributed throughout the oil phase, and likewise the lustrous effect of the mica pigment counteracts the matte effect of the wax gellant, creating a balanced result.

According to a further aspect of the present invention there is provided a means for counteracting the matte finish obtained when a fatty-alcohol gelled composition is applied topically to skin, particularly underarm skin, by incorporating dispersed within the oil phase at least 0.25% by weight, based on the composition, of a mica pigment.

### Detailed description of the present invention, including preferred embodiments.

The present invention relates to means for improving the visual appearance of skin to which an anhydrous gelled antiperspirant composition has been applied, the oil phase therein being gelled with a fatty alcohol.

The invention is applicable to compositions containing sufficient gellant that they are solid, that is to say retain their shape in the form of a bar or stick without lateral support. The hardness of solid sticks is commonly measured using a conventional penetrometer test in which a Seta needle weighing 50g and having a tip angle of 9° 10' +/- 15' is allowed to drop for 5 seconds from surface contact with the test material. Desirably, the needle penetrates less than 30mm, preferably less than 20mm and especially up to 15mm, by virtue of the concentration of gellant or gellant mixture employed to solidify the composition. When employing a fatty alcohol as gellant or as the major (i.e. >50% by weight) contributor to a gellant mixture, the probe rarely penetrates less than 7mm and often, the concentration of gellant is selected to achieve a penetration of from 10 to 15 mm. An alternative method of mesasuring hardness employs a Stable Micro Systems TA.XT2i Texture Analyzer and Texture Expert Exceeds™ software to generate the motion probile of a spherical probe employed in the method. A so measured hardness of at least 0.5N/mm² indicates a solid stick.

Herein, the fatty alcohol, which is linear, has a melting point in the range of from 55 to 75°C, and in many desirable embodiments, in the range of from 58 to 73°C. One or a blend of fatty alcohols can be employed, such as cetyl alcohol, stearyl alcohol, eicosyl alcohol and behenyl alcohol, or mistures of any two or more thereof. Commercial fatty alcohols, though nominally and predominantly one specified alcohol often comprise a minor fraction, such as up to 5 or 6% by weight in total, of homologues differing by 2, 4 or even 6 carbons.

The fatty alcohol provides in many suitable compositions from 65 to 85% by weight of the gellant mixture, and desirably at least 70%. By employing a co-gellant or cogellants, it is possible to modify the modify the hardness of the resultant product and to modify the composition during processing. It is especially desirable to employ gellant mixtures in which the fatty alcohol, and especially stearyl alcohol, constitutes from 11 to 20% by weight of the composition and especially from 12.5 to 18% by weight.

The co-gellant is desirably also one or more waxes, preferably having a melting point in the range of from 70 to 95°C and especially from 75 to 90°C. Such waxes are often selected from hydrocarbon waxes and ester waxes, that can be derived from natural sources or synthesised. Suitable hydrocarbon waxes include mineral wax, microcrystalline wax, Montana wax, and low molecular weight polyethylene, such as from 300 to 600 daltons. Suitable ester waxes can be derived from unsaturated natural oils, such as plant-originating triglyceride oils by hydrogenation and optionally dehydroxylation (where the substituent contains at least one hydroxyl group as in castor oil). Suitable ester waxes include caster wax, candelilla wax, carnauba wax, beeswax and spermeceti wax. Natural waxes such as beeswax include a range of different chemical classes. Synthetic esters often comprise aliphatic monoesters containing at least 30 carbons, and inc=deed may be isolated natural products such as beeswax, or be derived from them or be the same compounds.

It is highly desirable for the gellant mixture herein for a solid composition to contain from 2 to 8% by weight, particularly 3 to 6% by weight (based on the composition) of co-gellant and particularly of co-gellant having a melting point of from 75 to 85°C. The co-gerllant very suitably comprises a mixture of an esterwax, such as caster wax and a hydrocarbon wax, such as polyethylene of from 300 to 500 daltons, and conveniently in weight ratio of from 2:1 to 8:1.

The invention compositions herein, including particularly soft solid compositions, but also firm stick compositions advantageously comprise a silicone elastomer, by which is meant a crosslinked dimethicone. Elastomers tend to thicken oils, often by absorbing them and swelling. The elastomer is typically crosslinked by reacting a silicone hydride with an αω olefinically unsaturated dialkylene. The elastomer is advantageously present at a concentration of at least 0.1 % up to 8%, and especially from 0.5% to 5% by weight of the antiperspirant composition. Elastomers are commercially available, for example from Dow Corning Inc and Shinetsu, and typically are supplied in a carrier oil that is frequently a cyclomethicone.

The anhydrous compositions herein comprise at least one oil and usually a blend of oils. Herein an oil is a hydrophobic material that is liquid at 20°C (1 bar pressure). The weight proportion of oil is often up to 90%, and in preferred compositions up to 75%; is usually at least 40% and desirably at least 45% of the complete composition. An especially suitable range is from 50 to 65% by weight of the composition.

Oils employable herein commonly fall into two categories, namely silicone oils (sometimes called organo-silicone oils by virtue of organo-substitution) and non-silicone oils. Also, each of the categories can be divided into two types, namely volatile and non-volatile. The selection of the oils is at the discretion of the producer, and commonly comprises a mixture of at least one volatile oil and at least one non-volatile oil. Volatile herein indicates that the material generates a vapour pressure of at least 1 Pa and often from 10 to 2 kPa at 25°C. Selection of the balance between silicone and non-silicone oils, and between volatile and non-volatile oils is at the discretion of the producer of the cosmetic formulation, who would take into account, amongst other things, the sensory and other physical properties that he wished the resultant product to demonstrate and any constraints arising from choice of gellant and/or additional ingredients.

By his selection of silicone and/or non-silicone oils in varying proportions and volatile and non-volatile oils in varying proportions, compositions having different sensory properties can be obtained.

It is desirable to include volatile silicone because it gives a "drier" feel to the applied film after the composition is applied to skin. Volatile polyorganosiloxanes can be linear or cyclic or mixtures thereof. Preferred cyclic siloxanes include polydimethylsiloxanes and particularly those containing from 3 to 9 silicon atoms and preferably not more than 7 silicon atoms and most preferably from 4 to 6 silicon atoms, otherwise often referred to as cyclomethicones. Preferred linear siloxanes include polydimethylsiloxanes containing from 3 to 9 silicon atoms. The volatile siloxanes normally by themselves exhibit viscosities of below 10⁻⁵ m²/sec

(10 centistokes), and particularly above 10⁻⁷ m²/sec (0.1 centistokes), the linear siloxanes normally exhibiting a viscosity of below 5 x 10⁻⁶ m²/sec (5 centistokes). The volatile silicones can also comprise branched linear or cyclic siloxanes such as the aforementioned linear or cyclic siloxanes substituted by one or more pendant -0-Si(CH₃)₃ groups. Examples of commercially available volatile silicone oils include oils having grade designations 344, 345, 244, 245 and 246 from Dow Corning Corporation; Silicone 7207™ and Silicone 7158™ from Union Carbide Corporation; and SF1202™ from General Electric.

Often, the weight proportion of the volatile silicone oils is at least 10 or at least 20% of the total weight of silicone oils in the composition according to the present invention, and in many particularly suitable compositions, constitutes at least 70% and especially at least 85% by weight of the silicone oils. Based on the composition, the weight proportion of volatile oils and in particular volatile silicone oils is often selected in the range of from 20 to 40%, such as up to 35%.

The carrier oils employed in compositions herein can alternatively or additionally comprise one or more non-volatile silicone oils, which include polyalkyl siloxanes, polyalkylaryl siloxanes and polyethersiloxane copolymers. These can suitably be selected from dimethicone and dimethicone co-polyols. Commercially available non-volatile silicone oils include products available under the trademarks Dow Corning 556 and Dow Corning 200 series. Other non volatile silicone oils include that bearing the trademark DC704. Incorporation of at least some non-volatile silicone oil having a high refractive index such as of above 1.5, eg at least 10% by weight (preferably at least 25% to 100% and particularly from 40 to 80%) of the silicone oils can be beneficial in some compositions, such as where for example it is desirable to reduce visible deposits and/or produce a translucent composition by refractive index matching the dispersed particulate antiperspirant salt with the carrier oil (taking into account the influence of any humectant that forms a unitary phase with the carrier oil). Many non-silicone oils act as emollients. Any non-silicone oil provides the balance of the oils. The non-volatile carrier oils often constitute from 1 to 15%, such as 2 to 10% by weight of the silicone oils in the oil blend.

The liquid silicone oils can constitute up to 100% by weight of the water-immiscible liquid carrier oils, for example in many desirable embodiments, their weight proportion is selected in the range of at least 20 or 30% of the carrier oils, often in the range of at least 50% and in some especially preferred embodiments is selected in the range of at least 70% by weight. In various of the above and in other desirable embodiments according to the present invention, non-silicone oils constitute a large or major (>50%) weight proportion, or even up to 100% of the oil phase, for example at least 20 or 30%, particularly selected in the range of at least 50% and especially selected in the range of at least 70%.

### Non-silicone oils

The formulator of compositions according to the present invention can include one or more non-silicone oils, sometimes alternatively described as silicon-free hydrophobic or water-immiscible liquids, in addition to or instead of all or a fraction of the silicone oils mentioned hereinbefore. Such oils are, as indicated hereinbefore, liquid at 20°C at standard pressure, indeed are preferably liquid at 15°C and oils having a boiling point of at least 150°C are advantageous. The melting and boiling point data for chemical compounds is readily available in reference works such as the CRC Handbook of Chemistry and Physics published by CRC Press, often together with an indication of whether the compound is water soluble or miscible (hydrophobic is equivalent to water-immiscible). For any compound where such data is not available in the literature, it can be measured simply by any chemist using conventional techniques. Various non-silicone oils are volatile and many are non-volatile.

The non-volatile oils, when employed, other than non-volatile silicone oils are often selected from one or more of the following classes of organic compounds, namely, hydrocarbon oils, ester oils and ether oils.

Both volatile and non-volatile hydrocarbon oils are readily available. Volatile oils include, in particular, paraffins and isoparaffins containing an intermediate number of carbon atoms, for example chosen in the range of from 8 to 25 carbons, and often at least 10 carbons, depending on its molecular structure. However, non-ideal mixtures of hydrocarbons tend to have a higher volatility than would be suggested by the individual constituents, and melting and boiling points tend to increase with increasing molecular weight, so such numerical limits represent a guide and indeed there a diffuse transition to when hydrocarbons are clearly non-volatile. Volatile hydrocarbons can be employed instead of all or a proportion of the volatile silicone oils identified herein before. In many desirable invention formulations, the volatile hydrocarbon comprise from 0 to 20% by weight and especially from 0 to 10% by weight of the total oil blend. Compositions free from volatile hydrocarbons are particularly preferred.

Non-volatile aliphatic hydrocarbons are commonly selected from mineral oils, hydrogenated polydecene and hydrogenated polyisobutene. Non-volatile hydrocarbons can be incorporated to advantage on account of their desirable properties, since many, for example, exhibit emollient properties, the same or others have a low viscosity and by virtue of a mid-range refractive index, such as around 1.46 or 1.47, they generally assist in reducing the visibility of astringent antiperspirant salts when topically adhering to skin or clothing. Non-volatile hydrocarbon oils preferably are present in a proportion of from 0 to 50% w/w, in a number of advantageous embodiments from 0 to 10% w/w of the oils and in other advantageous embodiments of from 10 to 25% w/w of the oils. Suitable non-volatile hydrocarbons include hydrogenated polydecene and petrolatum, the latter commonly being a low melting point waxy material, such as in the region of 35 to 45°C.

Ester oils represent a particularly useful class of non-silicone oils, such as liquid aliphatic or aromatic esters. Typically such oils are regarded as non-volatile. The ester oils can be aliphatic, aromatic or contain both an aliphatic and an aromatic group. Many desirable aliphatic esters contain at least one long chain hydrocarbon group, for example from 8 to 25 carbons, derived from a monohydric alcohol or mono-carboxylic acid. Suitable aliphatic esters can be derived from monohydric alcohols such as selected from C₁ to C₂₀ alkanols esterified with a carboxylic acid selected from C₈ to C₂₂ mono alkanoic acid and C₆ to C₁₀ alkanedioic acids. Such esters include isopropyl myristate, lauryl myristate, isopropyl palmitate, diisopropyl sebacate and diisopropyl adipate. Other suitable ester oils include glyceride oils and in particular triglyceride oils derived from glycerol and fatty acids, sometimes olefinically unsaturated rather than saturated, containing at least 6 carbons and especially natural oils derived from unsaturated carboxylic acids containing from 16 to 20 and especially 18 carbons.

Suitable liquid aromatic esters or mixed aromatic/aliphatic esters are preferably derived from benzoic acid. Examples of such esters include suitable C₈ to C₁₈ alkyl benzoates or mixtures thereof, including in particular C₁₂ to C₁₅ alkyl benzoates. Many suitable benzoate esters are available under the trademark Finsolv. Other aromatic esters which can be contemplated for use herein comprise double aromatic inclusion. Benzyl benzoate, though feasible, is preferably substantially absent, such as at no more than 5%, and particularly no more than 3% or 1% by weight of the oil blend, and more particularly is excluded. Preferred double aromatic esters comprise a linear or branched alkyl chain, e.g. from 1 to 3 carbons, interposed between ester and/or ether substituted phenyl groups.

Aliphatic esters tend to exhibit an intermediate refractive index, and are therefore employed typically for their emollient properties. Aromatic esters tend to demonstrate a higher refractive index, such as around 1.49 to 1.50 and when double aromatic substitution is present, even an higher refractive index, rendering them particularly suitable for the preparation of translucent compositions containing a particulate astringent antiperspirant salt, and even salts containing zirconium.

Ester oils, be they aliphatic or aromatic desirably comprise from 0 to 60%, preferably from at least 10 or 15% up to 35 or 40% w/w of the oils, such as highly desirably 15 to 35% in various embodiments. It will recognised that the ester oils mentioned herein are commonly regarded as non-volatile and accordingly can be substituted for non-volatile silicone oils, for example silicone oils of similar refractive index, in whole or in part, at the discretion of the formulator.

In a number of highly desirable embodiments, the invention compositions contain a natural ester oil, either together with or absent any other ester oil. Such natural oils most desirably are jojoba oil or glyceride oils derived from one or more unsaturated C₁₈ fatty acids. In many instances, the oils comprise one or more triglycerides. The fatty acid residues in the oils can comprise, commonly, from one to three olefinic unsaturated bonds and often one or two. Whilst in many instances the olefinic bonds adopt the trans configuration, in a number of desirable products the bond or bonds adopt the cis configuration. If two or three olefinic unsaturated bonds are present, they can be conjugated. The fatty acid can also be substituted by an hydroxyl group. The natural oils employable herein desirably comprise one or more triglycerides of oleic acid, linoleic acid, linolenic acid or ricinoleic acid. Various isomers of such acids often have common names, including linolenelaidic acid, trans 7-octadecenoic acid, parinaric acid, pinolenic acid punicic acid, petroselenic acid and stearidonic acid. It is especially desirable to employ glycerides derived from oleic acid, linoleic acid or petroselenic acid, or a mixture containing one or more of them.

Natural oils containing one or more of such triglycerides include coriander seed oil for derivatives of petroselinic acid, impatiens balsimina seed oil, parinarium laurinarium kernel fat or sabastiana brasilinensis seed oil for derivatives of cis-parinaric acid, dehydrated castor seed oil, for derivatives of conjugated linoleic acids, borage seed oil and evening primrose oil for derivatives of linoleic and linolenic acids, aquilegia vulgaris oil for columbinic acid and sunflower oil, olive oil or safflower oil for derivatives of oleic acid, often together with linoleic acids. Other suitable oils are obtainable from hemp, which can be processed to derive stearadonic acid derivatives and maize corn oil. An especially convenient natural oil by virtue of its characteristics and availability comprises sunflower oil, ranging from those rich in oleic acid glycerides to those rich in linoleic acid glycerides, rich indicating that its content is higher than that of the other named acid.

The proportion of the natural oil such as triglyceride oil in the composition is often selected in the range of from 0.1 to 10% by weight of the carrier mixture, especially in the range of from at least 0.25% by weight and particularly at least 0.5%. Often, its weight proportion is selected in the range of up to 6% by weight and in many embodiments up to 4% of the carrier oils. A particularly convenient range comprises from 0.75 to 3% w/w of the carrier oils.

Ether oils represent further instances of suitable oils. Preferably, the ether oils contemplatable herein comprise liquid aliphatic ethers can be derived from a polyglycol, especially from polypropylene glycol, PPG, the latter preferably containing at least 3 mers, such as 3 to 20, with a monohydric alcohol. The monohydric alcohol often contains between 3 and 20 carbons. As the molecular weight of the PPG increases, so the chain length of the monohydric alcohol can decrease. Hence, for example, suitable ether oils can vary between a low molecular weight PPG with a long chain fatty alcohol, such as PPG-3 myristyl ether and a lower alkyl ethers of a higher molecular weight PPG, such as the ether named as PPG-14 butyl ether in the CTFA Handbook. Such ethers desirably constitute a weight proportion of from 0 to 30%, and preferably at least 5% of the oils, such as conveniently up to 20 or 15%.

In many desirable embodiments according to the present invention, the composition contains at least one ester oil and at least one ether, such as in a weight ratio of total ester: ether of from 5:1 to 1:5. In such or other embodiments, the composition desirably contains both a natural oil and an ether oil, for example selected in a range of weight ratios of from 1:5 to 1:20, and particularly from 1:9 to 1:15.

A further class of carrier oils, that can be contemplated herein comprises water-immiscible aliphatic alcohols that have a boiling point of above 100°C, including in particular branched chain aliphatic alcohols containing from 12 to 25 carbon atoms such as iso-stearyl alcohol and octyldocecanol. In such embodiments, such alcohol oils advantageously contribute from 10 to 50% by weight of the oil blend.

The total weight proportion of non-volatile oils in the composition is normally at least 10%, often at least 15% and desirably at least 20%. Its weight proportion is desirably not greater than 45%, often up to 40% and in many embodiments up to 35%.

The weight ratio of volatile to non-volatile oils in the composition is often selected in the range of from 2:1 to 1:2 and in many very desirable embodiments in the range of from 3:2 to 2:3, including particularly 5:4 to 4:5.

### Humectant

The compositions according to the present invention preferably further comprise a di or trihydric humectant. By including such a material, the demoisturising effect of an astringent antiperspirant salt can be counteracted to at least some extent. Accordingly, the humectant cooperates with the other ingredients, such as in particular, the mica pigment and if present the triglyceride oil and, again if present, any occlusive oil to provide not only a short term improvement in visual appearance of the skin to which the composition is applied, compared with applying compositions from which such beneficial ingredients are absent, but by prolonged use over for example weeks or months can improve the inherent quality of the skin, reducing the likelihood of visible imperfections and dry patches that can be instantly addressed by the incorporation of a smoothing aid into the antiperspirant composition.

The humectants contemplated herein are suitably propylene glycol, preferably glycerol and particularly preferably polyethylene glycol (PEG) having a molecular weight of from 200 to 600, such as from 250 to 500. As has been previously disclosed, propylene glycol and especially glycerol, are preferably pre-absorbed onto a particulate carrier, such as fumed silica to eliminate or at least drastically curtail their interaction with the antiperspirant active during manufacture of the invention composition, thereby avoiding or at least minimising grit formation. PEG humectant is especially suitable because it naturally avoids or at least greatly mitigates grit formation.

The weight proportion of the selected humectant, or mixture of humectants, is desirably at least 0.1 %, preferably at least 0.5% and especially at least 1% of the composition. Its proportion is attractively not more than 10% and in many desirable embodiments is up to 7.5%, and particularly up to 5% by weight of the composition.

An essential constituent of the invention compositions is the mica pigment. Such pigments are obtained by applying a thin coating of titanium dioxide, optionally with tin oxide and/or silicon dioxide to the surface of the mica, which often have adopted the physical form of platelets. In many instances, the coating further incorporates a minor fraction of a transition metal oxide, including in particular iron, chromium, copper or cobalt oxides or a combination of two or more of them. By incorporating the metal oxide, the resultant material exhibits a colour highlight that supplements the reflective character of the substrate mica. Such pigments are commonly called interference pigments. It is particularly to select an interference pigment that exhibits a highlight having a wavelength of below 550nm, particularly below 500nm. Many preferred pigments exhibit a highlight of wavelength greater than 400nm and particularly from 450nm. Suitable and/or preferred mica pigments and mica interference pigments are commercially available, such as various grades from Merck Inc under their trade mark Timiron.

Commonly at least 95% by weight of suitable mica pigments herein are sized below 60 µm, and usually have a mean (D-50) particle size of at least 95% by weight of particles below 25 µm, such as from 5 to 25 µm, with a mean between 7 and 13 µm. Such a pigment can conveniently be mixed with an other pigment of similar particle size and/or with a further pigment having a mean particle size of from 15 to 25 µm, such as in a ratio of from 1:3 to 3:1.

The invention compositions incorporate at least 0.25% by weight of the mica pigment and/or interference pigment that is distributed through the oil phase. Advantageously, in total, at least 0.5% by weight mica pigment and/or interference pigment is incorporated. Its weight proportion is often up to 4% and in many suitable embodiments is up to 2.5%.

In many embodiments herein, the linear fatty alcohol and interference pigment are present in a weight ratio of up to 80:1, and in preferred embodiments from 5:1 to 40:1 and particularly from 8:1 to 16:1.

Highly desirably, the mica pigment is present in a weight ratio to the humectant of from 3:2 to 1:3 and particularly from 1:1 to 1:2.

Very desirably, the mica pigment is present in a weight ratio to the natural oil of from 3:1 to 1:3 and particularly from 1:1 to 2:1.

The interference pigment cooperates with the non-volatile oil to enhance the visible appearance of the skin to which the composition has been topically applied. In many suitable inventions compositions herein, the non-volatile oil or blend of non-volatile oils, including any natural oil, is present in a weight ratio to the mica pigment of from 8:1 up to 40:1 and particularly from 12:1 up to 25:1.

A particular and particulate constituent of an antiperspirant composition is the antiperspirant active itself. The weight proportion of the astringent antiperspirant salt, in the composition or mixture if more than one salt is employed, is varied at the discretion of the manufacturer and normally in the range of from 5 to 30%, and in many desirable compositions at least 10 or 15%, such as up to 26% by weight.

Astringent salts employed herein are often selected from astringent aluminium, zirconium and mixed aluminium/zirconium salts, optionally complexed. Preferred aluminium, zirconium and aluminium/zirconium salts contain a halide, especially chloride and especially preferred salts are basic salts, which is to say a fraction of the halide within the empirical formula has been replaced by bound hydroxyl groups, such as at least half. Chlorohydrate salts are very highly desired. Aluminium halohydrates are usually defined by the general formula Al₂(OH)ₓQ_{y}.wH₂O in which Q represents chlorine, bromine or iodine, x is variable from 2 to 5 and x + y = 6 while wH₂O represents a variable amount of hydration. Aluminium chlorohydrate as made comprises a mixture of a number of different polymeric species in varying proportions, depending on the molar ratio of aluminium to chloride and the conditions employed during manufacture. All such mixtures are employable herein. It is especially desirable to employ what is commonly called activated aluminium chlorohydrate or enhanced activity aluminium chlorohydrate, sometimes abbreviated to AACH, in which the proportion of the more active species, such as Band III species (by a conventional chromatographic method) is higher by virtue of its method of manufacture. In one definition of activated, given in EP 6739, the material has greater than 20% Band III. Other methods of making AACH are given in EP 191628 and EP 451395. AACH is often made by recovery of an aluminium chlorohydrate from a dilute solution under strictly controlled reaction/maturing/dewatering/drying conditions. AACH is commercially available by name, or as activated or enhanced activity, from suppliers such as Reheis, Summit Research and B K Giulini.

Zirconium actives can usually be represented by the empirical general formula: ZrO(OH)_{2n-nz}B_{z.}wH₂O in which z is a variable in the range of from 0.9 to 2.0 so that the value 2n-nz is zero or positive, n is the valency of B, and B is selected from the group consisting of chloride, other halide, sulphamate, sulphate and mixtures thereof. Possible hydration to a variable extent is represented by wH₂O. Preferable is that B represents chloride and the variable z lies in the range from 1.5 to 1.87. In practice, such zirconium salts are usually not employed by themselves, but as a component of a combined aluminium and zirconium-based antiperspirant.

The above aluminium and zirconium salts may have coordinated and/or bound water in various quantities and/or may be present as polymeric species, mixtures or complexes. In particular, zirconium hydroxy salts often represent a range of salts having various amounts of the hydroxy group. Zirconium aluminium chlorohydrate may be particularly preferred.

Antiperspirant complexes based on the above-mentioned astringent aluminium and/or zirconium salts can be employed. The complex often employs a compound with a carboxylate group, and advantageously this is an amino acid. Examples of suitable amino acids include dl-tryptophan, dl-β-phenylalanine, dl-valine, dl-methionine and β-alanine, and preferably glycine which has the formula CH₂(NH₂)COOH.

It is highly desirable in some embodiments of the instant invention to employ complexes of a combination of aluminium halohydrates (especially chlorohydrates) and zirconium chlorohydrates together with amino acids such as glycine, which are disclosed in US-A-3792068 (Luedders et al). Certain of those Al/Zr complexes are commonly called ZAG in the literature. ZAG actives generally contain aluminium, zirconium and chloride with an Al/Zr ratio in a range from 2 to 10, especially 2 to 6, an Al/Cl ratio from 2.1 to 0.9 and a variable amount of glycine. Actives of this preferred type are available from Westwood, from Summit and from Reheis.

It is particularly preferred for the antiperspirant salts to be at least substantially free from aluminium sulphate, by which is meant that its weight proportion of the total weight of all antiperspirant salts present is less than 5%, especially less than 3% and particularly less than 1%. Total absence would be very suitable.

Other actives which may be utilised include astringent titanium salts, for example those described in GB 2299506A.

The particle size of the astringent antiperspirant salts feedstock often falls within the range of 0.1 to 100µm and particularly from at least 0.2µm. In many desirable products, the feedstock has at least 95% by weight of below 50µm with a mean particle size often from 2 to 30µm, in many instances from 2 to 10µm, and in certain other compositions from 10 to 25µm.

Where it is desired to form antiperspirant products which exhibit no greater than low visible deposits on topical application to skin, it is preferable to select feedstocks which comprise predominantly non-hollow solid particles, for example not than 5% or particularly than 2%, especially less than 1% of hollow spherical particles with diameter above 50µm. Hollow particles can be removed by use of suitable grinding apparatus and conditions.

The weight of particulate active antiperspirant salt herein commonly includes any water of hydration present.

In some especially desirable embodiments, the invention compositions comprise n addition to the mica pigment, a hair growth inhibitor. Advantageously, the hair growth inhibitor, such as palmatine, is present at a concentration of at least 0.001 % and often up to 0.01 %. The weight ratio of mica pigment to hair growth inhibitor is often selected in the range of from 200:1 to 1000:1, and particularly from 400:1 to 800:1. By employing a hair growth inhibitor in conjunction with the mica material, the composition not only improves the appearance of the skin on application, but the effect is maintained and increased with consecutive applications of the composition. For example, reduced hair growth reduces the need for shaving or plucking and therefore the skin is challenged less frequently, consequently with less redness or blotchiness arising that needs combating by the mica. The combination therefore generates healthier-looking skin than either constituent alone.

### Optional ingredients

Optional ingredients include wash-off agents, often present in an amount of at least 0.1 % w/w and advantageously at least 0.25% w/w up to 5% w/w to assist in the removal of the composition from skin or clothing. Often its weight percent is up to 1 %. Such wash-off agents are typically nonionic surfactants such as esters or ethers containing both a C₈ to C₂₂ alkyl moiety and a hydrophilic moiety which can comprise a polyoxyalkylene group (POE or POP) and/or a polyol, eg glycerol or sorbitol.

The compositions herein can incorporate one or more cosmetic adjuncts conventionally contemplatable for cosmetic solids. Such cosmetic adjuncts can include particulate skin feel improvers, such as talc or finely divided high melting point polyethylene, for example in an amount of up to about 10% and often in total in an amount of from 0.5 to 6%; inorganic particulates, preferably finely divided, such as fumed silica, for example in an amount of up to 2%; skin benefit agents such as allantoin, vitamins or lipids, for example in an amount of up to 5%; colours; preservatives such as butylhydroxytoluene, often in an amount of from 0.01 to 0.1 %; metal chelates, such as EDTA, for example in an amount of up to 1%; skin cooling agents, such a menthol and menthol derivatives, often in an amount of up to 2%, all of such percentages being by weight of the composition. A further optional constituent comprises a micro-fine aluminium oxide powder, such as Spectra-Al and/or a particulate polymethylmethacrylate such as Ganzpearl GMX-0810, in a concentration of up to 2.5% of either or both.

If desired, the composition can comprise a supplementary deodorant active, i.e. an active other than the antiperspirant salt. Suitable supplementary deodorant actives can comprise deodorant effective concentrations of deoperfumes, and/or microbicides, including particularly bactericides, such as chlorinated aromatics, including biguanide derivatives, of which materials known as Igasan DP300™ (triclosan), Tricloban™, and Chlorhexidine warrant specific mention. A yet another class comprises biguanide salts such as are available under the trade mark Cosmocil™. Supplementary deodorant actives are commonly employed at a concentration of from 0.1 to 5% by weight and often up to 1 % by weight of the composition.

A commonly employed adjunct is a perfume (fragrance), which is normally present at a concentration of from 0 to 4% and in many formulations from 0.25 to 2% or 2.5% by weight of the composition. The composition can contain as perfume, at the discretion of the producer, free fragrance, a profragrance, encapsulated fragrance or fragrance that is associated with a host substrate such as cyclodextrin, or a mixture of any two or more of such perfume options.

### Method of Manufacture

The compositions according to the present invention can be made conveniently in accordance with processes that have been employed hitherto using the same ingredients in the absence of the mica pigment employed herein to make firm sticks or semi solid compositions, respectively, with the proviso that the mica is distributed through the oil phase during the manufacture process by adequate mixing.

In general, a suitable general method of manufacture of a firm or semi-solid stick comprises the steps of
a) forming a mixture of an oil phase with an linear fatty alcohol gellant dispersed therein;
b) heating the mixture to an elevated temperature at which the gellant becomes molten or dissolved in the oil phase;
c) introducing mica pigment into the oil phase with localised mixing, such as shear mixing, or intensive mixing in a recycle loop.;
d) introducing particulate astringent antiperspirant salt into the oil phase, steps c) and d) severally being carried out before, after or simultaneously with step a or b);
e) introducing the resultant mixture into a dispenser, or for firm sticks alternatively into a mould and
f) cooling, or allowing said resultant mixture to cool, to below its setting temperature, at least part of this step optionally occurring before step e).

In a variant, at least part of the gellant or gellant mixture can be itself melted prior to introduction t-into the oil blend. The temperature to which the dispersed mixture is heated in step b) depends on the melting or dissolution point of the chosen gellant or if a combination of gellants is employed, the one having the highest melting or dissolution temperature. This temperature is commonly at least 60°C, and in many instances is in the range of from 70 to 95°C. Preferably, the gellants and oils are selected together such that the mixture in step b) need not be heated above 100°C, and in many highly desirable embodiments is heated to a temperature of from 75 to 85°C.

In the manufacture of a semi-solid composition, it can be advantageous to subject the cooling mixture to shear mixing through its quiescent setting temperature, so as to create a cream consistency rather than a firm stick.

In the context of the above method of manufacture, organic indicates the presence of carbon in a gellant that is solid at 40°C and melts or dissolves in the oil phase at a temperature of up to 150°C.

The order of introduction of the other ingredients into the oils is at the discretion of the manufacture. In some desirable embodiments, a mixture is formed comprising oils, gellant and mica before step b), and the astringent salt is introduced after step b), and especially after the mixture has been cooled or allowed to cool, for example to below 70°C. Post step b) introduction of the antiperspirant salt advantageously reduces the time for humectant, if also present, to bind salt particles before the composition attains its setting temperature, and the temperature reduction increases the viscosity of the composition to reduce the rate at which particles bump into each other.

It will be recognised that optional ingredients, if any, can be introduced at a convenient step in the process, such as hitherto employed or proposed in the absence of the humectant. Thus, any temperature sensitive ingredient is desirably introduced into the composition shortly before the dispenser is charged into the setting container, and preferably at a temperature within 10°C and especially within 5°C of the setting temperature.

### Dispensers

The compositions produced herein are suitable for dispensing from known cosmetic dispensers for firm sticks. Such dispensers commonly comprise a barrel, often of round or oval transverse cross section, having an opening at a first end through which the composition is dispensed and an elevator at an opposed second end that can be advanced towards the first end. The elevator fits within the barrel. Commonly, the first end can be covered with a cap, conveniently dimensioned to push it over the exterior of the barrel.

For firm sticks, the opening is the full cross section of the barrel. The elevator can be advanced by insertion of finger within the barrel or by co-operation between a threaded spindle and aperture in the elevator, the spindle being rotated by either an externally protruding rotor wheel or by a pawl arrangement. Suitable dispensers for firm sticks are described, for example in US 4232977, US4605330, WO09818695, WO09603899, WO09405180, WO09325113, WO09305678, EP1040445, US5997202, US5897263, US5496122, US5275496, US 6598767, US 6299369, or WO2002/03830.

The compositions of the present invention can be topically applied to skin, and particularly to underarm skin by extruding the composition in stick form above the top of the barrel and thereafter wiped across the skin surface, thereby depositing a fraction of the composition on the skin. The action can be repeated until the user considers that sufficient composition has been deposited, often in the region of 3 to 8 wipes per armpit.

It is particularly desirable to apply the composition shortly after the armpit has been washed or shaved, and preferably warmed, for example by the application of warm water. The skin at such times is particularly receptive for the application of a smoothing aid, such as the mica pigment to improve the visual appearance of rough skin, of pits, and wrinkles. The composition is thereafter left in place, conventionally, for a period of time commonly between 5 and 24 hours until it is washed off, usually using soap or a conventional shower gel, and water, for example applied using a flannel, loofah or sponge or even fingers. When seeking to inhibit perspiration, the weight of antiperspirant active applied per armpit is often in the range of from 0.15 to 0.5 grams.

Particular embodiments according to the present invention are described hereinafter by way of example only. Such embodiments can be modified by the skilled person in accordance with the foregoing detailed description of the invention.

### Comparison CA and Examples 1 to

In these Examples and comparison, firm sticks were prepared by the following general method:-

The oils, PEG, wash-off agent together with any silica, and the structurant waxes in the proportions summarised in Table 1 below were blended together and heated to approximately 85°C, by which time the wax structurants had melted to form an homogenous mixture. The mica pigment was introduced into the blend and agitated vigorously to ensure its widespread distribution throughout the mixture. The mixture was permitted to cool whilst maintaining stirring until its temperature had reached about 70°C, whereupon the antiperspirant was introduced followed by the fragrance. When the mixture reached about 62/63°C, it was bottom filed into conventional 50g dispensing canisters equipped with an intermediate skeletal platform and twist-up mechanism. When the material had solidified, the canister was inverted and the top was capped.

The following attributes of the compositions were then assessed by the following method and the results summarised in Table 1 below:-
- 10 Female panellists shaved their own axillae approx 12hrs before the study commenced and their underarms were assessed by an expert clinician in a well lit booth of a clinical test centre.
- Skin quality was scored on a 0 to 5 scale for tone, imperfections, radiance, visual smoothness and visual and the result recorded
- Test product was applied to the axillae by the assessor by wiping the stick 4 times across each axilla in a ventilated booth
- The Expert clinician assessment reassessed the skin quality attributes directly after product application on the same 0 to 5 scales in the same booth and the results recorded
- The difference between the score before and after application of the test product for each attribute was calculated and the average is summarised in Table 1 below, increases in tone, radiance and smoothness are recorded as positive as are decreases in imperfections.

Accordingly, the data summarised in Table 1 indicates the effectiveness of the test composition at improving the skin appearance.

The attributes were assessed by mapping onto the following scale (description of severity, coverage of axillae):-
- 0: none
- 1: light, up to 10%
- 2: mild, 11 to 25%
- 3: moderate, 26 to 50%
- 4: marked, 51 to 75%
- 5: severe, 76 to 100%

The attributes assessed were as follows:-

| | |
|---|---|
| Imperfections | tags, scars, chicken skin |
| Tone | unevenness, freckles, HP through to evenness |
| Radiance | dullness through healthy glow to brightness |
| Smoothness visible roughness to velvety, peachy | Smoothness visible roughness to velvety, peachy |

| *Delete this row before filing, please Vi* | **Std Dove5** **B-0 (CA02/2)** | **JW-OPS005** | **JW-OPS006** | **JWDior** **B-50.0001 (CA08)** | **GLO.00** **8007S (CA09)** | **GLO.00** **8015S (CA09)** | **DIOR1 W (CA10)** |
|---|---|---|---|---|---|---|---|
| **Comparison/Example No** | CA | Ex 1 | Ex 2 | Ex 3 | Ex 4 | Ex 5 | Ex 6 |
| **INCI Name** | %w/w | %w/w | %w/w | %w/w | %w/w | %w/w | %w/w |
| Cyclomethicone ¹ | 28.3 | 5 | 25 | 25.5 | 26.8 | 26.78 | 26.80 |
| C12-15 Alkyl Benzoate ² | 15 | 15 | 15 | 15 | 15 | 15.00 | 15.00 |
| PPG-14 Butyl Ether ³ | 9 | 9.5 | 9.5 | 9.5 | 9 | 9.00 | 9.00 |
| PEG-8 ⁴ | 2 | 1 | 1 | 2 | 2 | 2.00 | 2.00 |
| Dimethicone ⁵ | 1 | 1 | 1 | 1 | 1 | 1.00 | 1.00 |
| BHT | 0.05 | 0.05 | 0.05 | 0.05 | 0.05 | 0.05 | 0.05 |
| Silica ⁶ | 0.75 | 0 | 0 | 0.75 | 0.75 | 0.75 | 0.75 |
| Steareth-100 ⁷ | 0.45 | 0.5 | 0.5 | 0.5 | 0.45 | 0.45 | 0.45 |
| Polyethylene ⁸ | 0.75 | 1 | 1 | 1 | 0.75 | 0.75 | 0.75 |
| Hydrogenated Castor Oil ⁹ | 4 | 3.5 | 3.5 | 3.5 | 4 | 4.00 | 4.00 |
| Stearyl Alcohol ¹⁰ | 17 | 18 | 18 | 18 | 17 | 17.00 | 17.00 |
| Aluminium Zirconium Tetrachlorohydrex GLY ¹¹ | 20 | 20 | 20 | 20 | 20 | 20.00 | 20.00 |
| Helianthus Annuus Seed Oil ¹² | 0.5 | 0.5 | 0.5 | 0.5 | 0.5 | 0.50 | 0.50 |
| Parfum | 1.2 | 1.2 | 1.2 | 1.2 | 1.2 | 1.20 | 1.20 |
| Mica, Titanium Dioxide, Tin Oxide ¹³ | 0 | 0 | 0 | 0.25 | 0.25 | 0.25 | 0.25 |
| Mica, Titanium Dioxide ¹⁴ | 0 | 0 | 0 | 1.25 | 1.25 | 1.25 | 1.25 |
| Mica, Titanium Dioxide, Iron Oxide ¹⁵ | 0 | 0 | 0 | 0 | 0 | 0.02 | 0.00 |
| Mica, Titanium Dioxide ¹⁶ | 0 | 0.75 | 0.75 | 0 | 0 | 0.00 | 0.00 |
| Silicone Elastomer (10% in D5) ¹⁷ | 0 | 22 | 0 | 0 | 0 | 0.00 | 0.00 |
| PMMA ¹⁸ | 0 | 0 | 2 | 0 | 0 | 0.00 | 0.00 |
| Al oxide ¹⁹ | 0 | 2 | 2 | 0 | 0 | 0.00 | 0.00 |
| **Assessments** | | | | | | | |
| Tone | 1 | 5 | 6 | 6 | 8 | 7 | 7 |
| Radiance | 1 | 9 | 10 | 11 | 15 | 12 | 12 |
| Smoothness | 0 | 10 | 6 | 8 | 10 | 9 | 5 |

The assessments summarized in Table 1 above for Comparison CA show that topical application of a stick composition that is gelled mainly by stearyl alcohol, a representative fatty alcohol, does not improve the perceived tone, radiance or smoothness of axillary skin to any significant extent, in the absence of an aid that in included in the compositions of Examples 1 to 6. Such an aid is demonstrable a mica pigment, and particularly a mica interference pigment. In addition, the improvement in perceived skin quality can be enhanced by one or more additional constituents such as a silicone elastomer, PMMA and ultrafine aluminium oxide powder.

| | |
|---|---|
| **Notes** | |
| 1 Volatile Silicone DC245 | 11 REACH 908 |
| 2 Finsolv TN | 12 Sunflower Oil High Oleic |
| 3 Fluid AP | 13 Timiron Silk Blue |
| 4 Polyglykol 400 | 14 Timiron Star Luster MP-115 |
| 5 DC200/50 | 15 Colorona Oriental Beige |
| 6 Aerosil 200 | 16 Timiron MP-111 |
| 7 Brij 700P | 17 DC9045 |
| 8 Performalene 400 | 18 Ganzpearl GMX-0810 |
| 9 Castorwax MP80 | 19 Spectra-Al |
| 10 Lanette C18 DEO | |

## Claims

1. A cosmetic antiperspirant composition comprising from 5 to 30% by weight of an astringent antiperspirant salt, from 40 to 80% by weight of a continuous liquid phase comprising at least one water-immiscible oil and from 5 to 30% by weight of a gellant for the carrier liquid selected from fatty alcohol waxes, and containing at least 0.1 % by weight of at least one aliphatic dihydric or trihydric moisturiser having a molecular weight of not greater than 620 and/or at least 0.1 % by weight of a triglyceride oil, which composition further containing at least 0.25% by weight of a mica pigment dispersed within the continuous liquid phase.

2. A composition according to claim 1 which contains at least 0.5% by weight of the aliphatic dihydric or trihydric moisturiser.

3. A composition according to claim 1 or 2 in which the moisturiser is glycerol.

4. A composition according to claim 1 or 2 in which the moisturiser is polyethylene glycol having a molecular weight of from 200 to 500.

5. A composition according to any preceding claim which contains up to 5% by weight of the moisturiser.

6. A composition according to any preceding claim in which the mica pigment is present in a weight ratio to the moisturiser of from 3:2 to 1:3.

7. A composition according to any preceding claim which contains at least 0.5% by weight of the triglyceride oil.

8. A composition according to claim 7 which contains up to 4% by weight of the triglyceride oil.

9. A composition according to any preceding claim which the triglyceride oil is sunflower seed oil.

10. A composition according to any preceding claim in which the mica pigment is present in a weight ratio to the triglyceride oil of from 3:1 to 1:3.

11. A composition according to any preceding claim which contains up to 4% by weight of the mica pigment.

12. A composition according to claim 11 which contains from 0.5 to 2.5% by weight of the mica pigment.

13. A composition according to any preceding claim in which the mica pigment comprises a mica interference pigment.

14. A composition according to claim 13 in which the mica pigment has a highlight of wavelength less than 530nm.

15. A composition according to claim 14 in which the mica pigment has a highlight of wavelength less than 500nm.

16. A composition according to any preceding claim in which the fatty alcohol melts at between 55 and 73°C.

17. A composition according claim 16 in which the fatty alcohol comprises stearyl alcohol.

18. A composition according to any preceding claim in which the fatty alcohol constitutes from 65 to 85% by weight of the gellants.

19. A composition according to any preceding claim which contains at least 12.5% by weight of the fatty alcohol.

20. A composition according to any preceding claim in which the gellant comprises a co-gellant having a melting point of from 75 to 90°C.

21. A composition according to claim 20 which contains from 3 to 6% by weight of the co-gellant.

22. A composition according to claim 20 or 21 in which the co-gellant comprises a polyethylene wax, preferably having a molecular weight of from 300 to 600.

23. A composition according to claim 20 or 21 in which the co-gellant comprises caster wax.

24. A composition according to any preceding claim which contains at least one volatile silicone oil and at least one non-volatile oil.

25. A composition according to claim 24 which contains from 2 to 35% by weight of the volatile silicone oil.

26. A composition according to claim 24 which contains from 20 to 35% by weight of the non-volatile oil.

27. A composition according to claim 24 in which the weight ratio of the volatile to the non-volatile oil is selected in the range of from 2:3 to 3:2.

28. A composition according to any preceding claim which contains a hair growth inhibitor.

29. A composition according to claim 28 in which the hair growth inhibitor is palmatine.

30. A composition according to any preceding claim which contains a micro-fine aluminium oxide and/or a particulate polymethylmethacrylate.

31. A process for making a composition as described in any preceding claim which includes the step of dispersing the mica pigment in the oil, optionally in the presence of the gellant.

32. A method of simultaneously visually enhancing skin appearance and reducing perspiration in a localised region of skin, and particularly in the axilla, comprising topically applying to the skin an antiperspirant composition according to any of claims 1 to 30.

## Patentansprüche

1. Kosmetische schweißhemmende Zusammensetzung,
die folgendes aufweist:
5 bis 30 Gew.-% eines adstringierenden schweißhemmenden Salzes, 40 bis 80 Gew.-% einer kontinuierlichen flüssigen Phase, die zumindest ein mit Wasser nicht mischbares Öl und 5 bis 30 Gew.-% eines Geliermittels für die Trägerflüssigkeit aufweist, das aus Fettalkoholwachsen ausgewählt ist, und gegebenenfalls mindestens 0,1 Gew.-% von zumindest einem aliphatischen zweitwertigen oder dreiwertigen Feuchthaltemittel mit einem Molekulargewicht von nicht mehr als 620 und/oder mindestens 0,1 Gew.-% eines Triglyceridöls enthält, wobei die Zusammensetzung ferner mindestens 0,25 Gew.-% eines Glimmerpigments enthält, das in der kontinuierlichen flüssigen Phase dispergiert ist.

2. Zusammensetzung nach Anspruch 1,
die mindestens 0,5 Gew.-% des aliphatischen zweiwertigen oder dreiwertigen Feuchthaltemittels enthält.

3. Zusammensetzung nach Anspruch 1 oder 2,
wobei das Feuchthaltemittel Glycerol ist.

4. Zusammensetzung nach Anspruch 1 oder 2,
wobei das Feuchthaltemittel Polyethylenglycol mit einem Molekulargewicht von 200 bis 500 ist.

5. Zusammensetzung nach einem der vorstehenden Ansprüche,
die bis zu 5 Gew.-% des Feuchthaltemittels enthält.

6. Zusammensetzung nach einem der vorstehenden Ansprüche,
wobei das Glimmerpigment in einem Gewichtsverhältnis zum Feuchthaltemittel von 3:2 bis 1:3 vorliegt.

7. Zusammensetzung nach einem der vorstehenden Ansprüche,
die mindestens 0,5 Gew.-% des Triglyceridöls enthält.

8. Zusammensetzung nach Anspruch 7,
die bis zu 4 Gew.-% des Triglyceridöls enthält.

9. Zusammensetzung nach einem der vorstehenden Ansprüche,
wobei das Triglyceridöl Sonnenblumenöl ist.

10. Zusammensetzung nach einem der vorstehenden Ansprüche,
wobei das Glimmerpigment in einem Gewichtsverhältnis zum Triglyceridöl von 3:1 bis 1:3 vorliegt.

11. Zusammensetzung nach einem der vorstehenden Ansprüche,
die bis zu 4 Gew.-% des Glimmerpigments enthält.

12. Zusammensetzung nach Anspruch 11,
die 0,5 bis 2,5 Gew.-% des Glimmerpigments enthält.

13. Zusammensetzung nach einem der vorstehenden Ansprüche,
wobei das Glimmerpigment ein Interferenzpigment aus Glimmer aufweist.

14. Zusammensetzung nach Anspruch 13,
wobei das Glimmerpigment ein Maximum der Wellenlänge von weniger als 530 nm hat.

15. Zusammensetzung nach Anspruch 14,
wobei das Glimmerpigment ein Maximum der Wellenlänge von weniger als 500 nm hat.

16. Zusammensetzung nach einem der vorstehenden Ansprüche,
wobei der Fettalkohol bei 55 bis 73 °C schmilzt.

17. Zusammensetzung nach Anspruch 16,
wobei der Fettalkohol Stearylalkohol aufweist.

18. Zusammensetzung nach einem der vorstehenden Ansprüche,
wobei der Fettalkohol 65 bis 85 Gew.-% der Geliermittel ausmacht.

19. Zusammensetzung nach einem der vorstehenden Ansprüche,
die mindestens 12,5 Gew.-% des Fettalkohols enthält.

20. Zusammensetzung nach einem der vorstehenden Ansprüche,
wobei das Geliermittel ein Co-Geliermittel mit einem Schmelzpunkt von 75 bis 90 °C aufweist.

21. Zusammensetzung nach Anspruch 20,
die 3 bis 6 Gew.-% des Co-Geliermittels enthält.

22. Zusammensetzung nach Anspruch 20 oder 21,
wobei das Co-Geliermittel ein Polyethylenwachs, vorzugsweise mit einem Molekulargewicht von 300 bis 600, aufweist.

23. Zusammensetzung nach Anspruch 20 oder 21,
wobei das Co-Geliermittel Castorwachs aufweist.

24. Zusammensetzung nach einem der vorstehenden Ansprüche,
die zumindest ein flüchtiges Siliconöl und zumindest ein nicht-flüchtiges Öl enthält.

25. Zusammensetzung nach Anspruch 24,
die 2 bis 35 Gew.-% des flüchtigen Siliconöls enthält.

26. Zusammensetzung nach Anspruch 24,
die 20 bis 35 Gew.-% des nicht-flüchtigen Öls enthält.

27. Zusammensetzung nach Anspruch 24,
wobei das Gewichtsverhältnis zwischen dem flüchtigen und dem nicht-flüchtigen Öl im Bereich von 2:3 bis 3:2 ausgewählt ist.

28. Zusammensetzung nach einem der vorstehenden Ansprüche,
die einen Hemmstoff für das Haarwachstum enthält.

29. Zusammensetzung nach Anspruch 28,
wobei der Hemmstoff für das Haarwachstum Palmitin ist.

30. Zusammensetzung nach einem der vorstehenden Ansprüche,
die mikrofeines Aluminiumoxid und/oder ein teilchenförmiges Polymethylmethacrylat enthält.

31. Verfahren zum Herstellen einer Zusammensetzung nach einem der vorstehenden Ansprüche,
das den Schritt des Dispergierens des Glimmerpigments in dem Öl, gegebenenfalls in Gegenwart des Geliermittels aufweist.

32. Verfahren zum gleichzeitigen visuellen Verbessern des Aussehens der Haut und Vermindern der Schweißbildung in einem lokalisierten Hautbereich und insbesondere in der Achsel, das die topische Anwendung einer schweißhemmenden Zusammensetzung nach einem der Ansprüche 1 bis 30 auf der Haut aufweist.

## Revendications

1. Composition anti-transpirante cosmétique comprenant de 5 à 30 % en poids d'un sel anti-transpirant astringent, de 40 à 80 % en poids d'une phase liquide continue comprenant au moins une huile immiscible à l'eau et de 5 à 30 % en poids d'un gélifiant pour le liquide vecteur sélectionné parmi des cires d'alcool gras, et contenant facultativement au moins 0,1 % en poids d'au moins un hydratant dihydrique ou trihydrique aliphatique ayant une masse moléculaire de 620 ou moins et/ou d'au moins 0,1 % en poids d'une huile de triglycéride, laquelle composition contient en outre au moins 0,25 % en poids d'un pigment mica dispersé dans la phase liquide continue.

2. Composition selon la revendication 1, qui contient au moins 0,5 % en poids de l'hydratant dihydrique ou trihydrique aliphatique.

3. Composition selon la revendication 1 ou 2, dans laquelle l'hydratant est le glycérol.

4. Composition selon la revendication 1 ou 2, dans laquelle l'hydratant est le poly-(éthylèneglycol) ayant une masse moléculaire de 200 à 500.

5. Composition selon l'une quelconque des revendications précédentes, qui contient jusqu'à 5 % en poids de l'hydratant.

6. Composition selon l'une quelconque des revendications précédentes, dans laquelle le pigment mica est présent dans un rapport en poids par rapport à l'hydratant de 3 : 2 à 1 : 3.

7. Composition selon l'une quelconque des revendications précédentes, qui contient au moins 0,5 % en poids de l'huile de triglycéride.

8. Composition selon la revendication 7, qui contient jusqu'à 4 % en poids de l'huile de triglycéride.

9. Composition selon l'une quelconque des revendications précédentes, dans laquelle l'huile de triglycéride est une huile de tournesol.

10. Composition selon l'une quelconque des revendications précédentes, dans laquelle le pigment mica est présent dans un rapport en poids par rapport à l'huile de triglycéride de 3 : 1 à 1 : 3.

11. Composition selon l'une quelconque des revendications précédentes, qui contient jusqu'à 4 % en poids du pigment mica.

12. Composition selon la revendication 11, qui contient de 0,5 à 2,5 % en poids du pigment mica.

13. Composition selon l'une quelconque des revendications précédentes, dans laquelle le pigment mica comprend un pigment d'interférence mica.

14. Composition selon la revendication 13, dans laquelle le pigment mica a une surbrillance à une longueur d'onde de moins de 530 nm.

15. Composition selon la revendication 14, dans laquelle le pigment mica a une surbrillance à une longueur d'onde de moins de 500 nm.

16. Composition selon l'une quelconque des revendications précédentes, dans laquelle l'alcool gras fond entre 55 et 73 °C.

17. Composition selon la revendication 16, dans laquelle l'alcool gras comprend de l'alcool stéarique.

18. Composition selon l'une quelconque des revendications précédentes, dans laquelle l'alcool gras constitue de 65 à 85 % en poids des gélifiants.

19. Composition selon l'une quelconque des revendications précédentes, qui contient au moins 12,5 % en poids de l'alcool gras.

20. Composition selon l'une quelconque des revendications précédentes, dans laquelle le gélifiant comprend un cogélifiant ayant un point de fusion de 75 à 90 °C.

21. Composition selon la revendication 20, qui contient de 3 à 6 % en poids du cogélifiant.

22. Composition selon la revendication 20 ou 21, dans laquelle le cogélifiant comprend une cire de poly(éthylène), ayant de préférence une masse moléculaire de 300 à 600.

23. Composition selon la revendication 20 ou 21, dans laquelle le cogélifiant comprend de la cire de ricin.

24. Composition selon l'une quelconque des revendications précédentes, qui contient au moins une huile de silicone volatile et au moins une huile non volatile.

25. Composition selon la revendication 24, qui contient de 2 à 35 % en poids de l'huile de silicone volatile.

26. Composition selon la revendication 24, qui contient de 20 à 35 % en poids de l'huile non volatile.

27. Composition selon la revendication 24, dans laquelle le rapport en poids entre l'huile volatile et l'huile non volatile est sélectionné dans la plage de 2 : 3 à 3 : 2.

28. Composition selon l'une quelconque des revendications précédentes, qui contient un inhibiteur de croissance des poils.

29. Composition selon la revendication 28, dans lequel l'inhibiteur de croissance des poils est la palmatine.

30. Composition selon l'une quelconque des revendications précédentes, qui contient un oxyde d'aluminium microfin et/ou un poly-(méthacrylate de méthyle) particulaire.

31. Procédé de fabrication d'une composition telle que décrite dans l'une quelconque des revendications précédentes, qui inclut l'étape de dispersion du pigment mica dans l'huile, facultativement en présence du gélifiant.

32. Méthode permettant simultanément de renforcer visuellement l'aspect de la peau et de réduire la transpiration dans une région localisée de la peau, et en particulier dans l'aisselle, comprenant l'application topique sur la peau d'une composition anti-transpirante selon l'une quelconque des revendications 1 à 30.
